# EUROPEAN PATENT SPECIFICATION

(11) **EP 4 489 801 B1**
(45) Date of publication and mention of the grant of the patent: **22.07.2026**
(21) Application number: 23712067.0
(22) Date of filing: 08.03.2023
(51) Int. Cl.: A61L 2/10, A61L 2/24

(54) **SYSTEMS FOR IRRADIATING AN ENVIRONMENT WITH UV RADIATION**
SYSTEME ZUR BESTRAHLUNG EINER UMGEBUNG MIT UV-STRAHLUNG
SYSTEMES POUR IRRADIER UN ENVIRONNEMENT AVEC UN RAYONNEMENT UV

(30) Priority: 09.03.2022 GB 202203286; 07.04.2022 US 202263328412 P
(43) Date of publication of application: 15.01.2025
(73) Proprietor: Biocare UV Limited, Warrington Cheshire WA5 7ZB (GB)
(72) Inventor: HUMPHREYS, Michael Stafford, Warrington Cheshire WA5 7ZB (GB); LEATHERLAND, Adrian James, Warrington Cheshire WA5 7ZB (GB)
(74) Representative: Boult Wade Tennant LLP
(86) International application number: PCT/GB2023/050552
(87) International publication number: WO 2023/170417

(56) References cited:
- WO-A1-2022/005507
- ES-A1- 2 765 749
- US-A1- 2020 179 543
- US-A1- 2021 018 884

## Description

The present invention relates to the use of UV radiation for irradiating an environment, in particular, for the purpose of sterilisation of the environment.

As is well known, pathogens can be spread between occupants of an environment. This could be caused, for example, by workers in an office space sharing keyboards or other apparatus, or by contact between medical staff and various equipment in a hospital. It is known to use UV radiation for the purpose of sterilisation in certain environments. UV radiation is an effective way of destroying pathogens.

UV-C in particular is known to be a powerful bactericidal wavelength of radiation. However, UV-C has the potential to be dangerous to people, and so safety standards exist, limiting the allowable exposure of people to UV-C. Limiting the use of UV-C, on the other hand, limits its effectiveness for sterilising an environment.

WO2022/005507A1 describes a disinfection system including one or more light sources configured to emit ultraviolet light in an environment for human occupancy, and one or more occupancy sensors configured to acquire data indicative of occupancy of the environment for human occupancy. Intensity of the ultraviolet light emitted by the one or more light sources is controlled based on the data indicative of occupancy of the environment acquired by the one or more occupancy sensors.

US2020/179543A1 describes a disinfection device including a disinfection chamber having an interior volume and a radiation source coupled to the interior volume. The radiation source is arranged to emit disinfecting radiation into the interior volume when in operation. A disinfection program in the disinfection chamber is arranged to control the radiation source to emit the disinfecting radiation according to parameters determined based on at least one of a three dimensional model of the disinfection chamber and a three dimensional model of a target article to be disinfected.

There is therefore a need for systems and methods to use UV-C in an effective manner to sterilise an environment whilst ensuring safety for its occupants.

According to the invention there is provided a system according to claim 1.

The controller may control the emission of UV radiation from the one or more UV lamps based at least in part upon the irradiance map.

The system may comprise at least one sensor (and preferably a plurality of sensors) for measuring received UV radiation. The controller may update the irradiance map based on the measured UV radiation and/or the controller may control the emission of UV radiation from the one or more UV lamps based at least in part upon the UV radiation measurement.

The system comprises an occupancy sensor (and preferably a plurality of occupancy sensors) for outputting an occupancy signal. The controller is able to generate an occupancy map representing the use of the environment over time based on the occupancy signal, and analyse the occupancy map and the irradiance map to identify any risk zones within the environment for representing zones in which a modelled level of exposure to UV radiation is insufficient to have eradicated pathogens modelled to result from the measured level of occupancy.

The system is arranged to operate in one or more operating modes, the operating modes including a safe mode. In the safe mode the controller controls the emission of UV radiation so that the exposure at a first location within the environment to UV radiation over a first period of time is less than a threshold exposure.

In the safe mode the controller may control the emission of UV radiation for a first period of time so that there is an exposure at a first location within the environment to UV radiation and the exposure over the first period of time is less than a threshold exposure. In this way the safe mode is different to stopping the emission of UV radiation and is rather a mode in which there is an emission of UV radiation. However, the emission of UV radiation causes an exposure below a threshold exposure during the period in which the safe mode is activated.

According to the disclosure there is further provided a system for irradiating an environment with UV radiation, comprising one or more UV lamps for emitting UV radiation, at least one sensor for measuring a level of ozone in the environment, and a controller for controlling the emission of UV radiation from the one or more UV lamps based at least in part upon the measured level of ozone.

For a better understanding of the disclosure and to show how the same may be put into effect, reference will now be made, by way of example only, to the accompanying drawings in which:
Figure 1 shows a schematic representation of a first embodiment of a system in accordance with the disclosure
Figure 2a shows a schematic representation of a bathroom;
Figure 2b shows a schematic representation of an irradiance map for the bathroom of Figure 2a;
Figure 2c shows a schematic representation of an occupancy map for the bathroom of Figure 2a.

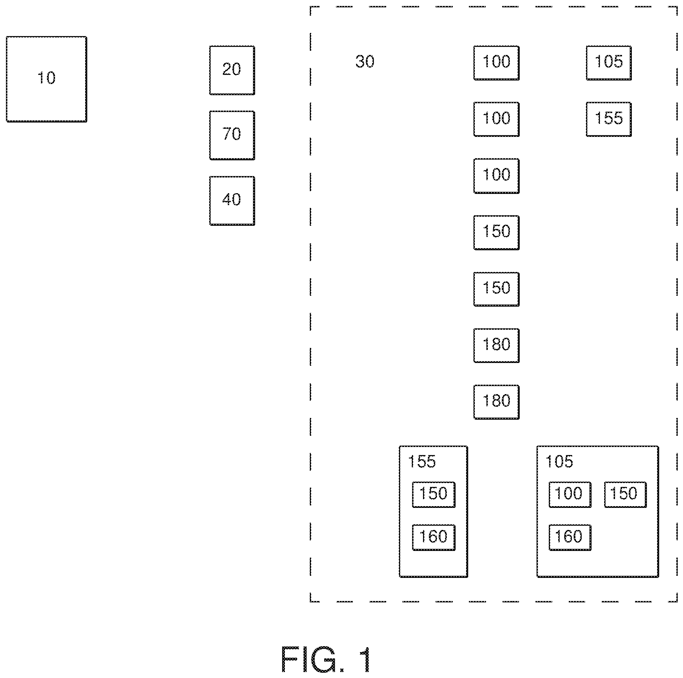

As can be seen in Figure 1, a first embodiment of a system for irradiating an environment 30 with UV radiation comprises: a plurality of UV lamps 100; and a controller 10 in communication with the UV lamps 100.

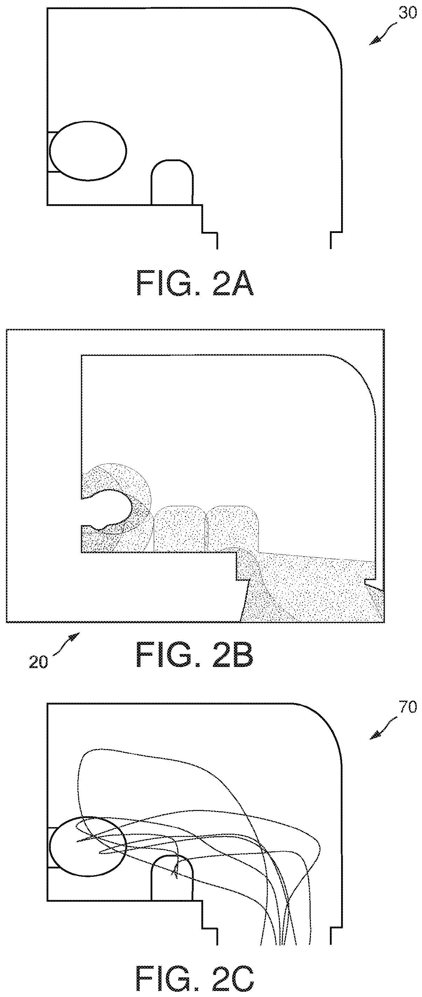

An example of an environment 30 is shown in Figure 2a. The depicted environment 30 is a bathroom.

The UV lamps 100 are arranged to emit UV radiation, preferably UV-C radiation, more preferably far-UV-C.

UV-C is defined as electromagnetic radiation having a wavelength in the range of from 200nm to 280nm. Far-UV-C is defined as electromagnetic radiation having a wavelength in the range of from 207nm to 222nm.

The UV lamps 100 may be mounted in fixed positions within the environment 30, for example, mounted in or to a ceiling of a room.

In preferable embodiments, the UV lamps 100 are arranged to emit UV radiation with a duty cycle that can be modulated by the controller 10. That is, the radiation emitted by the UV lamps 100 over a first period of time will be dependent upon the power output of the UV lamps 100 when on, and the ratio of time on to time off defining the duty cycle.

The controller 10 is arranged to control the emission of UV radiation from the UV lamps 100. That is, the controller 10 may control the duty cycle of the UV lamps 100 and/or the power output by the UV lamps 100 when they are switched on.

The controller 10 is arranged to store or access an irradiance map 20 (see Figure 2b, for example). The controller 10 may communicate with a remote server on which the irradiance map 20 is stored, or store a copy locally. The controller 10, UV lamps 100, and sensors 150, 180 (discussed below), and mobile units 105, 155 (discussed below) may all communicate wirelessly using known technologies such as WiFi, GPRS, and/or Bluetooth, etc.

The irradiance map 20 may be a 2D or 3D image representing the variation within the environment 30 of received UV radiation emitted by the UV lamps 100.

The environment 30 may be a room in a building. The system may be used in relation to multiple rooms. In which case, there may be a separate irradiance map 20 for each room, or there may be an irradiance map 20 spanning one or more rooms. In either case, the term environment 30 corresponds to the region corresponding to a single irradiance map 20 (that is, if there are two irradiance maps 20, there will be two environments 30).

The irradiance map 20 may define, for each location in the environment 30 a level of intensity of radiation received for a given power output of the UV lamps 100. Individually, the radiation emitted by each UV lamp 100 will be in accordance with an inverse square law, and so is predictable based on the geometry of the environment 30. An irradiance map 20 for a first environment 30 may differ from an irradiance map 20 for a second environment 30 because of the locations of the UV lamps 100 within the environments 30 and the shape and contents of the environments 30.

For example, a first room (an environment 30) may be rectangular and have a regular array of UV lamps 100 embedded in the ceiling. A second room may be square and have UV lamps 100 in two opposite upper corners of the room. The irradiance map 20 for the first room would differ from the irradiance map 20 for the second room.

Moreover, obstacles (such as furniture) within the environment 30 can vary the irradiance map 20. An obstruction of a UV lamp 100 will cast a "shadow" relative to that source of UV radiation. Such occlusions can add to the complexity of an irradiance map.

It can therefore be seen that the irradiance map 20 of an environment 30 is unlikely to show an even distribution of exposure to radiation (even in cases where the locations selected for the UV lamps 100 are intended to provide as even a distribution as possible).

The irradiance map 20 indicates how the radiation received from the UV lamps 100 varies across the environment 30. In some embodiments, the irradiance map 20 may be defined in a dimensionless manner that is independent of the power output of the UV lamps 100 such that it can be scaled in relation to the power output by the UV lamps 100.

In preferred embodiments, the irradiance map 20 indicates the expected power received from the UV lamps 100 at locations within the environment 30 when the UV lamps 100 are all switched on (i.e., independently from their duty cycle).

The irradiance map 20 may be generated using known illumination modelling methods. For example, the irradiance map 20 may be created using Optic Studio software from Zemax, LLC or DIALux from Dial GmbH.

It is possible to generate a 2D irradiance map 20, representing a plan view of the environment 30. This may be appropriate, for example, when all UV lamps 100 are mounted in a ceiling, since the irradiation is emitted downwardly. The locations in the plan view would represent the upper surfaces of any items included within the environment 30 that have been included in the modelling process. For example, if an empty room is modelled, the plan view might represent the floor, whereas if a room containing a table is modelled, the plan view might represent the floor around the table and the upper surface of the table.

In more complicated scenarios, a 3D irradiance map 20 may be appropriate. This might be because the UV lamps 100 are not all positioned at the same heights, or because the reflectivity of the walls of a room may be taken into account.

As will be appreciated, the level of complexity of modelling to generate the irradiance map 20 can vary greatly. However, the underlying process is the same, in that the irradiance map 20 provides an indication of locations that are subject to greater or lesser amounts of irradiation from the UV lamps 100.

The controller 10 may control the emission of UV radiation from the one or more UV lamps 100 based at least in part upon the irradiance map 20.

In particular, the system may be arranged to operate in a safe mode. In the safe mode the controller 10 controls the emission of UV radiation from the UV lamps 100 so that the environment may be safely occupied. That is, the occupants within the environment 30 will not be subjected to UV radiation exceeding a threshold exposure.

Specifically, in the safe mode, the controller 10 controls the emission of UV radiation from the UV lamps 100 so that the exposure at a first location within the environment 30 to UV radiation over a first period of time is less than a threshold exposure. The threshold exposure may be defined in units of joules per square metre, or other appropriate units of irradiance measurement.

The first period of time may be longer than the duty cycle. For example, the duty cycle might express the number of times per second that the UV lamps 100 turn on and off, while the first period of time might be greater than one second.

For example, the controller 10 may limit the UV radiation emitted from the UV lamps 100 to ensure that the first location is subjected to less than 22 joules per square metre over the time period from 9am to 5pm (e.g., a typical working day).

The first location may be selected as the location of the peak value in the irradiation map 20. In this case, at any location within the environment 30 the exposure to UV radiation over a first period of time is less than a threshold exposure.

The first location may be selected as the location in the irradiation map 20 that corresponds to the most occupied location within the environment 30 (discussed further below). In this case, at the highest occupancy location within the environment 30 the exposure to UV radiation over a first period of time is less than a threshold exposure.

For example, the controller 10 may determine from the irradiance map 20 the level of irradiance expected for the first location, and set the power output and/or duty cycle of the UV lamp 100 to set the exposure of the first location to a value less than the threshold exposure. When the first location corresponds to the point of greatest irradiance in the irradiance map 20, this will ensure that every location within the environment 30 is subjected to safe levels of UV radiation.

The performance characteristics of a UV lamp 100 can degrade over time or through use. The irradiance map 20 may be generated based on the assumption that the UV lamps 100 are newly manufactured and so in accordance with manufacturers specifications. As such, the accuracy of the irradiance map 20 can reduce over time. The system therefore is arranged to be able to re-calibrate in order to compensate for this variation.

The system may comprise at least one UV sensor 150 for measuring received UV radiation. The at least one UV sensor 150 may be fixed or mobile, or there may be a combination of fixed and mobile sensors 150. The UV sensor 150 is in communication with the controller 10controller 10.

In preferred embodiments, the controller 10 may update the irradiance map 20 based on the UV radiation measurement. Alternatively, or additionally, the controller 10 may simply incorporate in the control of the emission of UV radiation from the one or more UV lamps 100 a factor determined by the UV radiation measurement.

A single measurement in an environment 30 is sufficient to vary the irradiance map 20 to compensate for a general degradation of all of the UV lamps 100 for that environment 30. This may be appropriate in most cases, since the usage of each UV lamp 100 will be identical, and so the degradation through use would be similar.

In other embodiments having multiple UV lamps 100, it may be preferred to compensate for the differences in degradation between UV lamps 100. In these cases, either a plurality of distributed fixed UV sensors 150 or a mobile UV sensor 150 can make multiple UV radiation measurements at different locations in the environment 30, and regression analysis such as a least squares fit may be applied to determine the degradation in performance of each UV lamp 100.

In some embodiments the system comprises a fixed UV sensor 150 within the environment 30. The location in the irradiance map 20 corresponding to the fixed UV sensor 150 is known. Therefore, the controller 10controller 10 may be recalibrated based on a comparison of the UV radiation measurement by the fixed UV sensor 150 with the value expected for that location based on the irradiance map 20. That is, the controller 10 may control the emission of UV radiation from the one or more UV lamps 100 based at least in part upon the irradiance map 20 and the UV radiation measurement.

The same recalibration may be made with UV sensors 150 mounted on a sensing mobile unit 155. When the UV sensors 150 are mobile, there is also provided a location determination module 160 for providing location information indicating the location of the sensing mobile unit 155.

The location determination module 160 may be provide as part of the sensing mobile unit 155 to which a UV sensor 150 is mounted. In this case, the location determination module could comprise an inertial measurement unit, for example.

Alternatively, or additionally, the location determination module 160 can be external to the sensing mobile unit 155, such as one or more cameras in the environment 30 that visually track the position of the sensing mobile unit 155.

The sensing mobile unit 155 wirelessly transmits a UV radiation measurement to the controller 10controller 10. The UV radiation measurement is associated with the location information indicating the location of the sensing mobile unit 155 at the time the measurement was taken. The association may be carried out by the sensing mobile unit 155 or the controller 10controller 10.

The controller 10controller 10 may control the position of the sensing mobile unit 155, or it may be autonomous.

In some cases, the controller 10controller 10 may direct the sensing mobile unit 155 to take a measurement at a particular location within the environment 30. In other cases, the sensing mobile unit 155 may store a path to follow to take measurements at predefined locations within the environment 30.

The controller 10controller 10 may select a location for measurement at least in part based on the irradiance map 20. For example, the controller 10controller 10 may direct the sensing mobile unit 155 to make a measurement with UV sensor 150 at a location in the environment 30 corresponding to the peak value in the irradiation map 20.

The controller 10controller 10 may select a location for measurement at least in part based on an occupancy map 70 (discussed below). For example, the controller 10controller 10 may direct the sensing mobile unit 155 to make a measurement with the UV sensor 150 at a location in the environment 30 corresponding to the most occupied location in the occupancy map 70.

In some cases, the sensing mobile unit 155 may autonomously navigate through the environment 30, making UV radiation measurements using at least one UV sensor 150, the UV radiation measurements being associated with a location at which the measurement was taken.

For example, for a building with a plurality of rooms (such as a hospital), each room may define an environment 30. The sensing mobile unit 155 may drive on a path through the various rooms, either following a fixed path, or navigating around obstacles. The sensing mobile unit 155 periodically and/or at particular locations and/or when instructed by the controller 10controller 10 will use UV sensor 150 to measure received UV radiation. The UV radiation measurement will be provided to the controller 10controller 10 to be used in combination with the location at which the measurement was taken to re-calibrate the controller 10 and/or update the irradiance map 20.

The controller 10 can provide a timing signal to the sensing mobile unit 155 to ensure that the measurement is synchronised with the period of activation of the UV lamps 100 at a particular location (the "on" part of the duty cycle), or the measurement can be made over a period of time sufficient to capture a plurality of on-off cycles of the UV lamps 100.

The system may comprise an occupancy sensor 180 for outputting an occupancy signal. The occupancy sensor 180 is in communication with the controller 10.

In some embodiments, the occupancy signal may simply indicate the presence or absence of an occupant within the environment 30 (for example, the occupancy sensor 180 may be a passive infrared sensor). In such embodiments, the operating modes may include an unrestricted mode that may be triggered when the occupancy signal indicates that no occupant is present in the environment 30.

In the unrestricted mode, the controller 10 can control the UV lamps 100 to produce greater levels of radiation than in the safe mode, because no occupant will be exposed to the radiation. For example, in the unrestricted mode, the controller 10 controls the emission of UV radiation so that the exposure at the first location within the environment 30 to UV radiation over the first period of time is more than the first threshold.

In some cases, the controller 10 may switch from the unrestricted mode to the safe mode in response to the occupancy signal from the occupancy sensor 180 indicating that an occupant has entered the environment 30 (this switch may occur immediately). Similarly, the controller 10 may switch from the safe mode to the unrestricted mode in response to the occupancy signal from the occupancy sensor 180 indicating that no occupants remain in the environment 30 (this switch may occur after a period of time has elapsed following the occupancy sensor 180 indicating that the environment 30 is unoccupied).

In further embodiments, the occupancy sensor 180 may be used to generate an occupancy map 70 (see Figure 2c, for example), indicating a level of occupancy of the environment 30 over time. This can be referred to as a measured level of occupancy. The occupancy sensor 180 may be formed in a variety of ways known in the art, from camera-based systems for visual tracking to an array of sensors in the floor for sensing the weight of occupants.

The occupancy map 70 preferably covers the same area and is in registration with the irradiance map 20. For example, the occupancy map 70 may be an image (2D or 3D), with the locations in the image representing the proportion of time that the corresponding location in the environment 30 was occupied by an occupant. In this way, a map of usage of the environment 30 may be generated.

The controller 10 may generate or receive an occupancy map 70 representing the use of the environment over time based on the occupancy signal from one or more occupancy sensors 180. Since pathogens may be carried into the environment 30 by occupants, the occupancy map 70 provides an estimate of the risk of encountering a pathogen in locations within the environment 30. In contrast, the irradiance map 20 provides an estimate of the exposure to pathogen-destroying radiation of those locations within the environment 30.

The controller 10 may be configured to analyse the occupancy map 70 and the irradiance map 20 to identify any risk zones within the environment 30. This may be done, for example, by generating a risk map indicating an estimated risk of encountering a pathogen at locations within the environment 30.

A risk zone may be defined as a zone in which a level of exposure to UV radiation is insufficient to have eradicated pathogens expected to result from the occupancy of the zone. Alternatively, a risk zone may be defined as a zone in which a level of exposure to UV radiation is insufficient to have reduced pathogens expected to result from the occupancy of the zone to a level considered safe (i.e., below an acceptable threshold level).

Mathematical modelling may be employed to model an expected level of pathogens at each location within the environment 30 over a time period (for example, by integrating the occupancy of a location over the time period).

This may be done for one or more of a list of pathogens. For example, when setting up the system, or when interacting with the system, a user may define a list of pathogens. For example, a computer terminal in communication with the controller 10 may display a list of pathogens for selection by the user.

Pathogen data 40 may be stored in a manner accessible by the controller 10. Pathogen data 40 may represent the behaviour of pathogens over time in dependence upon UV radiation exposure. That is, pathogen data 40 may indicate the rate at which each of one or more pathogens multiply over time when not subjected to UV radiation and represent the rate decrease in number over time when subjected to UV radiation of different intensities. For example, pathogen data 40 may have been obtained from empirical studies into the use of UV radiation to destroy pathogens.

The controller 10 may use the pathogen data 40 to estimate the risk at different locations in the environment 30 based on the occupancy map 70 (which may indicate estimated sources of pathogens at particular times and locations) and the irradiance map 20 (which may indicate the exposure of those introduced pathogens to UV radiation).

The controller 10 may store a time series of duty cycle and/or power outputs associated with each UV lamp 100 over the time period, and combine this with the irradiance map 20, to provide an estimate of exposure of each location within the environment 30 over that time period.

The time period could be selectable at any level of granularity, for example, from minutes to one or more days.

The controller 10 can use the occupancy map 70 and the irradiance map 20 to identify the risk zones within the environment 30. Alerts may be generated based upon the risk zones.

For example, in a hospital, the occupancy map 70 may indicate that an area of the hospital has been occupied for an extended period of time (which would imply that the system has operated in safe mode and so the environment 30 would have been exposed to a level of radiation below the exposure threshold). It may be the case that the irradiance map 20 indicates that the highest occupied locations in the environment 30 were subject to the highest levels of radiation and so remain generally low risk. Alternatively, it may be the case that the locations in the environment 30 subjected to lower levels of irradiation have had high occupancy, indicating that they are risk zones.

The controller 10 may issue an alert, for example to the hospital staff, indicating that the area is a risk zone. The hospital staff may temporarily evacuate the area. The occupancy sensors would then indicate that the area is unoccupied and so the system may switch to the unrestricted mode to expose the likely level of pathogens to higher levels of sterilising UV radiation. In a further example, a hospital manager may be presented with a display representing the risk of user-selectable pathogens in each area of the hospital in real-time, such as a map of the hospital that shows risk varying over time based on occupancy and irradiation.

In some embodiments, a UV mobile unit 105 may be provided. The UV mobile unit 105 may comprise a UV lamp 100 for emitting UV radiation. The location determination modules 160 may also provide location information indicating the location of the UV mobile unit 105, again either as part of the UV mobile unit 105, or externally of the UV mobile unit 105.

The controller 10 may be arranged to direct the UV mobile unit 105 to an identified risk zone for providing additional radiation at the risk zone using the UV lamp 100 of the UV mobile unit 105. In some cases, the sensing mobile unit 155 will be a UV mobile unit 105. That is, the sensing mobile unit 155 may have both a UV sensor 150 and a UV lamp 100, and carry out both the sensing described above and additionally be directable by the controller 10 to risk zones for additional irradiation of those zones.

The controller 10 may be arranged to direct the UV mobile unit 105 to an identified risk zone for providing additional radiation at the risk zone using the UV lamp 100 of the UV mobile unit 105.

Generally, or because a risk zone has been identified, the controller 10 may direct the UV mobile unit 105 based on the irradiance map 20. Specifically, if the irradiance map 20 indicates an area that receives insufficient radiation, this may be supplemented by the UV lamp 100 of the UV mobile unit 105.

Generally, or because a risk zone has been identified, the controller 10 may increase the emission of UV radiation. This can be accomplished by increasing the electrical power supplied to the one or more UV lamps 100 or changing a duty cycle of the UV lamps 100. The emission of UV radiation may be increased in the identified risk zones, or in locations where the irradiance map 20 indicates that the area has received insufficient radiation. This can be achieved by increasing the electrical power supplied to one or more UV lamps 100 in the vicinity of the identified risk zones.

Whereas the system has been mainly described with reference to a single environment 30 with a corresponding irradiance map 20 and occupancy map 70, with a single UV mobile unit 105 and a single sensing mobile unit 155, in fact, the invention is applicable to multiple environments 30 which can each have or can share a plurality of UV sensors 150, occupancy sensors 180, and mobile units 105, 155. Moreover, each environment may have a dedicated controller 10, or multiple environments 30 may share a controller 10. When multiple controllers 20 are provided, they may communicate with one another and/or with a central server. Furthermore, one or more mobile units 105, 155 may move within and between multiple environments 30 under the control of one or more controllers 20.

A first embodiment of a method of irradiating an environment 30 with UV radiation comprises: modelling the radiation emitted from one or more UV lamps 100 to generate an irradiance map 20 for representing the variation within the environment 30 of received UV radiation emitted by the UV lamps 100; and controlling the emission of UV radiation from the one or more UV lamps 100 based at least in part upon the irradiance map 20.

The method may comprise selecting an operating mode from a plurality of operating modes that comprise a safe mode and an unrestricted mode.

Operating in the safe mode comprises controlling the UV lamps 100 so that the exposure at a first location within the environment 30 to UV radiation over a first period of time is less than a threshold exposure.

Operating in the unrestricted mode comprises controlling the UV lamps 100 so that the exposure at the first location within the environment 30 to UV radiation over the first period of time is more than the first threshold. In the unrestricted mode, the UV lamps 100 may be activated to emit radiation at full power.

The method may comprise selecting one of the safe mode or the unrestricted mode based on a determination of whether the environment 30 is occupied.

The method may also comprise, in the safe mode, measuring received UV radiation at a location within the environment 30 to provide a UV radiation measurement, and controlling the UV lamps 100 based at least in part upon the UV radiation measurement.

The method may also comprise measuring received UV radiation at a location within the environment 30 to provide a UV radiation measurement and updating the irradiance map based at least in part upon the UV radiation measurement.

In preferred embodiments, the method comprises directing a mobile unit 105, 155 based on the irradiation map.

The method may further comprise generating an occupancy map 70 for the environment 30 to represent the use of the environment 30 over time by occupants. The mobile units 105, 155 may be directed based on the occupancy map 70.

The method may further comprise identifying risk zones within the environment 30 using the occupancy map 70 and the irradiance map 20, and may involve generating an alert based on the identification of a risk zone.

One or more UV mobile units 105 may be directed to an identified risk zone and irradiate the risk zone using the UV lamp 100 of the UV mobile unit 105.

In a further embodiment, the system comprises at least one sensor for measuring a level of ozone in the environment 30. It has been realised that UV radiation can create ozone which is hazardous to the health of occupants of the environment 30. In this embodiment, the controller 10 is arranged to control the emission of UV radiation from the one or more UV lamps 100 based at least in part upon the measured level of ozone. This is done to ensure that the level of ozone does not exceed an ozone threshold level. Above the ozone threshold level it may be unsafe for the occupants of the environment 30. For example, when the at least one sensor detects that the level of ozone is approaching, equals and/or exceeds the ozone threshold level, the emission of UV radiation from the one or more UV lamps 100 is reduced and/or stopped.

Since ozone is gaseous and spreads throughout the environment 30, this embodiment does not require the use of the irradiance map 20 and is rather based on the level of ozone across the environment 30. If the environment 30 comprises more than one isolated area wherein ozone cannot freely pass from one area to another, then each area can be considered as a separate environment 30 for this purpose. That is, the level of ozone in each area is measured separately (for example, one or more ozone sensor is provided for each area) and the controller 10 controls the emission of UV radiation from the one or more UV lamps 100 located in that area based at least in part upon the measured level of ozone in that area. For example, if the environment 30 comprises a plurality of rooms, then level of ozone in each room is measured separately and the controller 10 controls the emission of UV radiation from the one or more UV lamps 100 located in that room based at least in part upon the measured level of ozone in that room.

The use of the at least one sensor for measuring a level of ozone may combined with any of the features described above, such as the irradiance map 20, the safe mode; the UV sensor 150 for measuring received UV radiation; the mobile unit 155; the occupancy sensor 180 and the occupancy map 70. In such an embodiment, the controller 10 is arranged to control the emission of UV radiation from the one or more UV lamps 100 based on several different sensors. Therefore, the emission of UV radiation from the one or more UV lamps 100 may be reduced and/or stopped when either the level of ozone is approaching, equals and/or exceeds the ozone threshold level or the safe mode is active, for example.

## Claims

1. A system for irradiating an environment (30) with UV radiation, comprising:
one or more UV lamps (100) for emitting UV radiation;
an occupancy sensor (180) for outputting an occupancy signal; and
a controller (10), wherein the controller (10) is:
arranged to store or access an irradiance map (20), the irradiance map (20) for representing the variation within the environment (30) of received UV radiation emitted by the UV lamps (100); and
for controlling the emission of UV radiation from the one or more UV lamps (100) based at least in part upon the irradiance map (20),
wherein:
the system is arranged to operate in one or more operating modes, the operating modes including a safe mode;
in the safe mode the controller (10) controls the emission of UV radiation so that the exposure at a first location within the environment (30) to UV radiation over a first period of time is less than a threshold exposure;
the controller (10) generates an occupancy map (70) representing the use of the environment (30) over time based at least in part on the occupancy signal; and
the controller (10) uses the occupancy map (70) and the irradiance map (20) to identify any risk zones within the environment (30) for representing zones in which a modelled level of exposure to UV radiation is insufficient to have reduced pathogens modelled to result from the measured level of occupancy to below an acceptable threshold.

2. The system of claim 1, further comprising a sensor (150) for measuring received UV radiation to provide a UV radiation measurement, wherein the controller (10) controls the emission of UV radiation from the one or more UV lamps (100) based at least in part upon the UV radiation measurement.

3. The system of claim 2, wherein the controller (10) updates the irradiance map (20) based at least in part upon the UV radiation measurement.

4. The system of claim 2 or claim 3, further comprising a mobile unit (155) to which the sensor (150) is mounted, the system having a location determination module (160) for providing location information indicating the location of the mobile unit (155), wherein the mobile unit (155) transmits a UV radiation measurement to the controller (10), the UV radiation measurement being associated with the location information.

5. The system of claim 4, wherein the controller (10) directs the mobile unit (155) based on the irradiation map (20).

6. The system of any preceding claim, wherein:
the operating modes including an unrestricted mode;
in the unrestricted mode the controller controls the emission of UV radiation so that the exposure at the first location within the environment (30) to UV radiation over the first period of time is more than the first threshold; and
the controller (10) selects one of the safe mode or the unrestricted mode based at least in part on the occupancy signal.

7. The system of claim 4, wherein the controller (10) directs the mobile unit (155) based on the occupancy map (70).

8. The system of any preceding claim, wherein the controller (10) generates an alert based on the identification of a risk zone.

9. The system of any preceding claim, further comprising a mobile unit (105) having a UV lamp (100) for emitting UV radiation, wherein the controller (160) directs the mobile unit (105) to an identified risk zones and the mobile unit (105) irradiates the risk zone using the UV lamp (100) of the mobile unit (105).

10. The system of any of any preceding claim, wherein the controller (10) increases the emission of UV radiation in the identified risk zones using the one or more UV lamps (100).

## Patentansprüche

1. System zur Bestrahlung einer Umgebung (30) mit UV-Strahlung, umfassend:
eine oder mehrere UV-Lampen (100) zum Emittieren von UV-Strahlung;
einen Belegungssensor (180) zum Ausgeben eines Belegungssignals; und
eine Steuereinheit (10), wobei die Steuereinheit (10) ausgelegt ist:
eine Bestrahlungsstärkekarte (20) zu speichern oder darauf zuzugreifen, wobei die Bestrahlungsstärkekarte (20) zur Darstellung der Variation der von den UV-Lampen (100) emittierten UV-Strahlung innerhalb der Umgebung (30) dient; und
zur Steuerung der Emission von UV-Strahlung aus der einen oder den mehreren UV-Lampen (100) zumindest teilweise auf der Grundlage der Bestrahlungsstärkekarte (20),
wobei:
das System so ausgelegt ist, dass es in einem oder mehreren Betriebsmodi arbeitet, wobei die Betriebsmodi einen sicheren Modus beinhalten;
im sicheren Modus die Steuereinheit (10) die Emission von UV-Strahlung so steuert, dass die Exposition gegenüber UV-Strahlung an einer ersten Stelle innerhalb der Umgebung (30) über einen ersten Zeitraum kleiner als ein Expositionsschwellenwert ist;
die Steuereinheit (10) eine Belegungskarte (70), die die Nutzung der Umgebung (30) im Zeitverlauf darstellt, zumindest teilweise auf der Grundlage des Belegungssignals erstellt; und
die Steuereinheit (10) die Belegungskarte (70) und die Bestrahlungsstärkekarte (20) verwendet, um etwaige Risikozonen innerhalb der Umgebung (30) zu identifizieren, die Zonen darstellen, in denen ein modelliertes Expositionsniveau gegenüber UV-Strahlung nicht ausreicht, um die modellierte Reduktion von Krankheitserregern, die sich aus dem gemessenen Belegungsniveau ergibt, unter einen akzeptablen Schwellenwert zu senken.

2. System nach Anspruch 1, ferner umfassend einen Sensor (150) zum Messen empfangener UV-Strahlung, um eine UV-Strahlungsmessung bereitzustellen, wobei die Steuereinheit (10) die Emission von UV-Strahlung aus der einen oder den mehreren UV-Lampen (100) zumindest teilweise auf der Grundlage der UV-Strahlungsmessung steuert.

3. System nach Anspruch 2, wobei die Steuereinheit (10) die Bestrahlungsstärkekarte (20) zumindest teilweise auf der Grundlage der UV-Strahlungsmessung aktualisiert.

4. System nach Anspruch 2 oder Anspruch 3, ferner umfassend eine mobile Einheit (155), an der der Sensor (150) montiert ist, wobei das System ein Standortbestimmungsmodul (160) zum Bereitstellen von Standortinformationen aufweist, die den Standort der mobilen Einheit (155) angeben, wobei die mobile Einheit (155) eine UV-Strahlungsmessung an die Steuereinheit (10) übermittelt, wobei die UV-Strahlungsmessung mit den Standortinformationen verknüpft ist.

5. System nach Anspruch 4, wobei die Steuereinheit (10) die mobile Einheit (155) auf der Grundlage der Bestrahlungskarte (20) lenkt.

6. System nach einem vorstehenden Anspruch, wobei:
die Betriebsmodi einen uneingeschränkten Betriebsmodus beinhalten;
im uneingeschränkten Modus die Steuereinheit die Emission von UV-Strahlung so steuert, dass die Exposition gegenüber UV-Strahlung an der ersten Stelle innerhalb der Umgebung (30) während des ersten Zeitraums den ersten Schwellenwert überschreitet; und
die Steuereinheit (10) eines von dem sicheren Modus oder dem uneingeschränkten Modus zumindest teilweise auf der Grundlage des Belegungssignals auswählt.

7. System nach Anspruch 4, wobei die Steuereinheit (10) die mobile Einheit (155) auf der Grundlage der Belegungskarte (70) lenkt.

8. System nach einem vorstehenden Anspruch, wobei die Steuereinheit (10) eine Warnung auf der Grundlage der Identifizierung einer Risikozone erzeugt.

9. System nach einem vorstehenden Anspruch, ferner umfassend eine mobile Einheit (105) mit einer UV-Lampe (100) zum Emittieren von UV-Strahlung, wobei die Steuereinheit (160) die mobile Einheit (105) zu einer identifizierten Risikozone lenkt und die mobile Einheit (105) die Risikozone unter Verwendung der UV-Lampe (100) der mobilen Einheit (105) bestrahlt.

10. System nach einem vorstehenden Anspruch, wobei die Steuereinheit (10) die Emission von UV-Strahlung in den identifizierten Risikozonen unter Verwendung der einen oder mehreren UV-Lampen (100) erhöht.

## Revendications

1. Système d'irradiation d'un environnement (30) par rayonnement UV, comprenant :
une ou plusieurs lampes UV (100) pour émettre un rayonnement UV ;
un capteur d'occupation (180) pour émettre un signal d'occupation ; et
un dispositif de commande (10), dans lequel le dispositif de commande (10) est :
agencé pour stocker ou accéder à une carte d'irradiance (20), la carte d'irradiance (20) permettant de représenter la variation, au sein de l'environnement (30), du rayonnement UV reçu émis par les lampes UV (100) ; et
pour commander l'émission de rayonnement UV d'une ou plusieurs lampes UV (100) en se basant au moins en partie sur la carte d'irradiance (20),
dans lequel :
le système est agencé pour fonctionner dans un ou plusieurs modes de fonctionnement, les modes de fonctionnement incluant un mode sécurisé ;
en mode sécurisé, le dispositif de commande (10) commande l'émission de rayonnement UV de sorte que l'exposition à un premier emplacement dans l'environnement (30) au rayonnement UV sur une première période de temps soit inférieure à un seuil d'exposition ;
le dispositif de commande (10) génère une carte d'occupation (70) représentant l'utilisation de l'environnement (30) au fil du temps, en se basant au moins en partie sur le signal d'occupation ; et
le dispositif de commande (10) utilise la carte d'occupation (70) et la carte d'irradiance (20) pour identifier les zones à risque dans l'environnement (30) afin de représenter les zones dans lesquelles un niveau d'exposition modélisé aux rayonnements UV est insuffisant pour réduire les agents pathogènes modélisés résultant du niveau d'occupation mesuré en dessous d'un seuil acceptable.

2. Système selon la revendication 1, comprenant en outre un capteur (150) pour mesurer le rayonnement UV reçu afin de fournir une mesure du rayonnement UV, dans lequel le dispositif de commande (10) commande l'émission de rayonnement UV à partir d'une ou plusieurs lampes UV (100) en se basant au moins en partie sur la mesure du rayonnement UV.

3. Système selon la revendication 2, dans lequel le dispositif de commande (10) met à jour la carte d'irradiance (20) en se basant au moins en partie sur la mesure du rayonnement UV.

4. Système selon la revendication 2 ou 3, comprenant en outre une unité mobile (155) sur laquelle le capteur (150) est monté, le système présentant un module de détermination de localisation (160) pour fournir des informations de localisation indiquant la localisation de l'unité mobile (155), dans lequel l'unité mobile (155) transmet une mesure de rayonnement UV au dispositif de commande (10), la mesure de rayonnement UV étant associée aux informations de localisation.

5. Système selon la revendication 4, dans lequel le dispositif de commande (10) dirige l'unité mobile (155) en fonction de la carte d'irradiation (20).

6. Système selon une quelconque revendication précédente, dans lequel :
les modes de fonctionnement incluent un mode non restreint ;
en mode non restreint, le dispositif de commande gère l'émission de rayonnement UV de sorte que l'exposition au rayonnement UV au premier emplacement dans l'environnement (30) pendant la première période de temps soit supérieure au premier seuil ; et
le dispositif de commande (10) sélectionne l'un parmi le mode sécurisé ou le mode non restreint en fonction au moins en partie du signal d'occupation.

7. Système selon la revendication 4, dans lequel le dispositif de commande (10) dirige l'unité mobile (155) en fonction de la carte d'occupation (70).

8. Système selon une quelconque revendication précédente, dans lequel le dispositif de commande (10) génère une alerte basée sur l'identification d'une zone à risque.

9. Système selon une quelconque revendication précédente, comprenant en outre une unité mobile (105) dotée d'une lampe UV (100) pour émettre un rayonnement UV, dans lequel le dispositif de commande (160) dirige l'unité mobile (105) vers une zone à risque identifiée et l'unité mobile (105) irradie la zone à risque à l'aide de la lampe UV (100) de l'unité mobile (105).

10. Système selon l'une quelconque des revendications précédentes, dans lequel le dispositif de commande (10) augmente l'émission de rayonnement UV dans les zones à risque identifiées à l'aide d'une ou plusieurs lampes UV (100).
